# EUROPEAN PATENT SPECIFICATION

(11) **EP 3 090 736 B1**
(45) Date of publication and mention of the grant of the patent: **29.05.2019**
(21) Application number: 14877461.5
(22) Date of filing: 30.07.2014
(51) Int. Cl.: A61K 9/48

(54) **AQUEOUS COMPOSITION FOR HARD CAPSULE, AND HARD CAPSULE PRODUCED USING SAME**
WÄSSRIGE ZUSAMMENSETZUNG FÜR HARTKAPSEL UND DAMIT HERGESTELLTE HARTKAPSEL
COMPOSITION AQUEUSE POUR GÉLULE ET GÉLULE FABRIQUÉE À PARTIR DE CELLE-CI

(30) Priority: 31.12.2013 KR 20130168262
(43) Date of publication of application: 09.11.2016
(73) Proprietor: LOTTE Fine Chemical Co., Ltd., Ulsan, 44714 (KR)
(72) Inventor: SON, Jin Ryul, Incheon 406-736 (KR); CHUN, Jeong Hee, Incheon 401-731 (KR); JEONG, Ji Seon, Incheon 405-835 (KR)
(74) Representative: Kador & Partner PartG mbB
(86) International application number: PCT/KR2014/006993
(87) International publication number: WO 2015/102197

(56) References cited:
- WO-A1-2006/082842
- WO-A1-2014/088177
- JP-A- 2009 504 630
- KR-A- 20010 033 693
- KR-A- 20080 106 160
- KR-A- 20110 135 876
- US-A- 3 493 407
- US-A- 5 264 223
- US-A1- 2004 022 845
- NING Z ET AL: "Composite useful for preparing vegetal empty hard capsule, comprises preset amount of coagulator, loading agent, plasticizer, ethanol as foam killer, and water", WPI / THOMSON,, vol. 2008, no. 66, 30 April 2008 (2008-04-30), XP002754260, -& CN 101 167 705 A (HAIFENG SHI [CN]) 30 April 2008 (2008-04-30)

## Description

### [Technical Field]

The present invention relates to an aqueous composition for a hard capsule, a method for preparing the same, and a hard capsule produced using the aqueous composition. More particularly, the present invention relates to an aqueous composition for a hard capsule which includes a gelation agent and a gelation auxiliary agent whose concentrations are reduced to appropriate levels, thereby improving a dissolution rate of a hard capsule, and a hard capsule produced using the aqueous composition.

### [Background Art]

Capsules used for medical supplies and health functional foods are generally produced using gelatin or cellulose ether as a base.

Gelatin capsules have advantages such as high industrial productivity and price competitiveness, but also have a drawback in that the plasticity may be lost and the impact resistance may be significantly deteriorated when a moisture concentration is less than or equal to 10% by weight. Also, since the current use of gelatin has been limited due to problems such as bovine spongiform encephalopathy, capsules produced using a plant material such as cellulose ether rather than the gelatin have come into the spotlight.

Generally, methods for producing a hard capsule may be mainly divided into two methods such as a Hot pin process and a cold pin process, depending on gel characteristics.

First of all, the Hot pin process uses a property of a cellulose ether solution being gelled when the cellulose ether solution is heated to a high temperature, and thus is a method of dipping a high-temperature mold pin into a cellulose ether solution maintained at a temperature higher than room temperature and thermally gelling the solution coated onto the pin through heat of the pin to produce a hard capsule.

As such a conventional method, Patent Document 1 (Registered US Patent No. 2,526,683) discloses a method of producing a medicinal methyl cellulose capsule using a dip coating method. Such a method includes dipping a mold pin, which has been preheated at 40 to 85°C, into a methyl cellulose composition maintained at a temperature lower than a temperature at which gelation starts to occur, recovering the pin, placing the pin in an oven at a temperature higher than a gelation point, and drying the resulting film. When the high-temperature pin is dipped into the composition, the composition is gelled on a surface of the pin. When the pin is recovered, a film made from a gelled liquid and having a predetermined thickness is formed on the pin. Subsequently, the pin is generally rotated at an angle of 180° to a vertical posture, and typically placed in an oven to be dried. Then, the dried capsule is peeled off, cut into pieces of a certain size, and fitted with a cap and a body portion. However, methyl cellulose is insoluble in water having a temperature of less than 37°C.

Next, the cold pin process includes heating a solution prepared from gelatin which is gelled at room temperature, or a hydroxypropyl methyl cellulose (HPMC) solution including a compound, such as carrageenan, which is gelled at room temperature (a gelation agent), followed by aging the solution at a constant high temperature, dipping a cold mold pin into the solution to coat the mold pin with a predetermined amount of the solution, removing the mold pin from the solution, immediately exposing the solution coated on the mold pin to cold blown air having a temperature of approximately 20°C to form a gel, and drying the gel to produce a capsule. The gelation agent used in the cold pin process has been generally used to produce capsules with metal ions such as potassium, calcium, and sodium to improve a gel-forming ability of the gelation agent. However, when a capsule to which a gelation agent such as carrageenan is added is orally administered, the gelation agent has a problem in that it re-reacts with metal salts present in gastric or intestinal juices to enhance a binding strength between capsule components, which makes it impossible to disintegrate the capsule. That is, an initial dissolution rate of the hard capsule is shown to be low due to an ionic characteristic of the gelation agent, and the hard capsule may exhibit other dissolution characteristics, depending on each medium.

To solve these problems, as a plan to reduce concentrations of the gelation agent and a gelation auxiliary agent, Patent Document 2 (Registered US Patent No. 5,756,123) discloses a capsule film composition which includes 18 to 28 parts by weight of HPMC, 0.01 to 0.1 parts by weight of carrageenan as the gelation agent, and 0.05 to 0.6 parts by weight of potassium or calcium ions as the gelation auxiliary agent. However, the even though dissolution rate is partially improved by such a method, the method has a drawback in that the quality of a capsule may be lowered due to the deteriorated moldability of the hard capsule. Patent Document 3 (CN 101 167 705 A) discloses in the abstract an aqueous composition for preparing a hard capsule comprising a coagulator (2-10), loading agent (40-60), plasticizer (1-20), ethanol (2-15), and water (10-100) The coagulator is carrageenan. The loading agent is hydroxypropyl methylcellulose, ethyl cellulose, carboxymethyl cellulose, tylose or hydroxyethylcellulose. The plasticizer is glycerin. Embodiment 6 discloses a composition composed of 0.16 kg calcium chloride (a gelation auxiliary agent, 0.3%), 2.4 kg plasticizer (glycerol, 4.54%), 19.2 kg water, sorbitol (1kg), τ-carrageenan (a gelation agent, 2kg, 3.8%), HPMC (25kg, 47.4%), ethanol 3kg (5.7%). All compounds are heated to 95°C, then the mixture is cooled to 53°C.

### [Prior-Art Documents]

### [Patent Documents]

(Patent Document 1) US2526683 B
(Patent Document 2) US5756123 B
(Patent Document 3) CN101167705 A

### [Disclosure]

### [Technical Problem]

Therefore, the present invention is designed to solve the problems of the prior art, and therefore it is an object of the present invention to provide an aqueous composition for a hard capsule capable of producing a hard capsule having an excellent dissolution characteristic when concentrations of a gelation agent and a gelation auxiliary agent are reduced to appropriate levels.

It is another object of the present invention to provide a hard capsule produced using the aqueous composition for a hard capsule.

### [Technical Solution]

To solve the above problems, according to an aspect of the present invention, there is provided an aqueous composition for a hard capsule which includes a water-soluble cellulose ether, an alcohol, and water, wherein the aqueous composition for a hard capsule further includes a gelation agent at greater than 0 parts by weight and not more than 0.5 parts by weight, and a gelation auxiliary agent at 0.3 to 0.6 parts by weight, based on 100 parts by weight of the aqueous composition for a hard capsule.

The at least one water-soluble gum selected from the group consisting of carrageenan, gellan gum, xanthan gum, and pectin is used as the gelation agent, and at least one selected from the group consisting of potassium chloride, potassium acetate, and calcium chloride is used as the gelation auxiliary agent.

Preferably, a concentration of the water-soluble cellulose ether may be in a range of 10 to 25% by weight, and a concentration of the alcohol may be in a range of 5 to 30% by weight, based on 100% by weight of the aqueous composition for a hard capsule.

According to another aspect of the present invention, there is provided a hard capsule produced using the aqueous composition for a hard capsule.

### [Advantageous Effects]

According to the present invention, when amounts of a gelation agent and a gelation auxiliary agent added to an aqueous composition for a hard capsule are reduced to appropriate levels, an initial dissolution rate of a hard capsule produced from the aqueous composition for a hard capsule can be improved, and a problem of the hard capsule exhibiting other dissolution characteristics depending on each medium can be solved.

Also, according to the present invention, when an alcohol is included in the aqueous composition, solubility of the water-soluble cellulose ether can be improved, and thus an aqueous composition for a hard capsule in which moldability of a hard capsule is not deteriorated even when concentrations of the gelation agent and the gelation auxiliary agent are reduced can be provided.

### [Description of Drawings]

FIG. 1 is a graph illustrating dissolution rates of hard capsules produced in Examples 1 to 5 and Comparative Examples 1 and 3 in water over time.
FIG. 2 is a graph illustrating dissolution rates of the hard capsules produced in Examples 1 to 5 and Comparative Examples 1 and 3 in gastric juice (pH 1.2) over time.

### [Best Mode]

The present invention is directed to an aqueous composition for a hard capsule which includes a water-soluble cellulose ether, an alcohol, and water. Here, the aqueous composition for a hard capsule further includes a gelation agent at greater than 0 parts by weight and not more than 0.5 parts by weight, and a gelation auxiliary agent at 0.3 to 0.6 parts by weight, based on 100 parts by weight of the aqueous composition for a hard capsule.

A concentration of the gelation agent is greater than 0 parts by weight and not more than 0.5 parts, preferably 0.3 to 0.5 parts by weight, based on 100 parts by weight of the aqueous composition for a hard capsule. In this case, the concentration of the gelation agent refers to a concentration (exclusive) of the gelation agent, based on the total weight of the aqueous composition. When the concentration of the gelation agent is greater than 5 parts by weight, a dissolution rate of a hard capsule may be lowered, and thus a viscosity of the aqueous composition for a hard capsule may excessively increase, which makes it difficult to produce capsules. Also, the hard capsule produced using the aqueous composition may be easily broken due to low elongation at break and high brittleness, and thus qualities of the capsule may be deteriorated. A water-soluble gum selected from the group consisting of carrageenan, gellan gum, xanthan gum, and pectin is used as a gelation agent.

A concentration of the gelation auxiliary agent is in a range of 0.3 to 0.6 parts by weight, based on 100 parts by weight of the aqueous composition for a hard capsule. In this case, the concentration of the gelation auxiliary agent refers to a concentration (exclusive) of the gelation agent, based on the total weight of the aqueous composition. When the concentration of the gelation auxiliary agent is less than 0.3 parts by weight, the concentration of the gelation auxiliary agent is insufficient to improve a gelling ability of the gelation agent, which makes it impossible to obtain an aqueous composition for a hard capsule having excellent moldability. On the other hand, when the concentration of the gelation auxiliary agent is greater than 0.6 parts by weight, workability and product qualities may be degraded due to an increase in viscosity of the aqueous composition for a hard capsule. At least one selected from the group consisting of potassium chloride, potassium acetate, and calcium chloride is used as the gelation auxiliary agent.

Meanwhile, the water-soluble cellulose ether is a main component of the aqueous composition for a hard capsule. Such a water-soluble cellulose ether is derived from cellulose that is a plant material, and thus has an advantage in that it is harmless to humans. In this specification, the term "cellulose ether" refers to a cellulose derivative obtained by etherifying a hydroxyl group of cellulose using an etherifying agent.

A concentration of the water-soluble cellulose ether may preferably be in a range of 10 to 25% by weight, based on the weight of the aqueous composition for a hard capsule. When the concentration of the water-soluble cellulose ether is in this range, bubbles may be easily removed since the resin composition may have a proper viscosity, and thus a capsule having a proper thickness may be obtained. At least one selected from the group consisting of hydroxypropyl methyl cellulose (HPMC), hydroxyethyl methyl cellulose (HEMC), and methyl cellulose (MC) may be used as the water-soluble cellulose ether.

The alcohol serves to aid in liquefying (that is, dissolving) the water-soluble cellulose ether in the aqueous composition for a hard capsule. Specifically, when the water-soluble cellulose ether is added to water having a low temperature (20 to 30°C), only a portion of the water-soluble cellulose ether coming in direct contact with the water is dissolved, but the other portion which does not come in direct contact with the water aggregates to form clusters. On the other hand, when the water-soluble cellulose ether is added to water having a high temperature (40 to 70°C), even a portion of the water-soluble cellulose ether coming in direct contact with the water tends not to be easily dissolved. However, the alcohol is mixed with water to form an aqueous alcohol solution, and the water-soluble cellulose ether is easily dissolved in an aqueous alcohol solution having a low temperature (20 to 30°C) as well as an aqueous alcohol solution having a high temperature (40 to 70°C).

A concentration of the alcohol may preferably be in a range of 5 to 30% by weight, based on the weight of the aqueous composition for a hard capsule. When the concentration of the alcohol is in this range, solubility of the cellulose ether may be enhanced, and an evaporation rate of the alcohol during production of the capsule may become appropriate, and thus a smooth capsule film having no wrinkles may be obtained. At least one selected from the group consisting of ethanol, methanol, isopropanol, and butanol may be used as the alcohol.

The aqueous composition for a hard capsule according to one exemplary embodiment of the present invention may further include a plasticizer at 0.05 to 5.0 parts by weight, both exclusive, based on 100 parts by weight of the aqueous composition. When the concentration of the plasticizer is in this range, a hard capsule having a high elongation at break may be obtained. At least one selected from the group consisting of glycerol, sorbitol, propylene glycol, and polyethylene glycol may be used as the plasticizer.

Also, in the preparation of the aqueous composition, an emulsifying agent for improving moldability of the capsule may be additionally added to water to prepare the aqueous composition. At least one selected from the group consisting of sodium lauryl sulfate (SLS), and a sugar ester (SE) may be used as the emulsifying agent. In particular, SLS may significantly improve a capsule forming ability. A concentration of the emulsifying agent may be in a range of 0.01 to 1.0 parts by weight, and preferably, 0.05 to 0.5 parts by weight, both exclusive, based on 100 parts by weight of the aqueous composition. When the concentration of the emulsifying agent is in this range, a decrease in drying property of the aqueous composition coated onto a mold pin may be realized, and thus a capsule having excellent moldability and good quality and exhibiting high stability with which the occurrence of gastroenteric troubles upon drug taking is suppressed may be obtained.

Hereinafter, one example of the method for preparing an aqueous composition for a hard capsule will be described in detail.

The method for preparing an aqueous composition according to one exemplary embodiment of the present invention may include mixing water and an alcohol to prepare an aqueous alcohol solution (S1), heating the aqueous alcohol solution (S2), adding cellulose ether to the heated aqueous alcohol solution to prepare a cellulose ether solution (S3), aging the cellulose ether solution (S4), and adding a gelation agent and a gelation auxiliary agent to the resulting solution (S5).

In the step S2, the heating of the aqueous alcohol solution may be performed at a temperature ranging from room temperature (20 to 30°C) to a temperature of 40 to 70°C. This step S2 is done to readily disperse the water-soluble cellulose ether in the aqueous alcohol solution so that the water-soluble cellulose ether is readily dissolved in the aqueous alcohol solution without aggregation in the step S3. When the heating temperature is in this range, an aqueous composition for a hard capsule having high capsule moldability and exhibiting a minimal increase in energy cost caused by inevitable heating may be obtained without solidifying a gelation agent and a gelation auxiliary agent to be described below.

The step S3 may be performed by slowly adding the water-soluble cellulose ether into the heated aqueous alcohol solution while stirring (for example, at 300 rpm).

The aging of the cellulose ether solution (S4) may be performed at a temperature of 40 to 70°C for 2 to 12 hours. When the water-soluble cellulose ether is completely dissolved by the aging, the aqueous composition has the following advantages: first, has a shorter production time; second, exhibits high homogeneity and uniform viscosity, and has no the layor separation of the solution even when stored for a long period of time; third, has a constant viscosity maintained for all production units; fourth, has high capsule moldability since insoluble products (for example, cellulose ether) suppressing the functions of a gelation agent and an gelation auxiliary agent do not exist; fifth, has high miscibility between cellulose ether and the gelation agent (and the gelation auxiliary agent), resulting in a decrease in the amount of the added gelation agent and gelation auxiliary agent; and, sixth, has high filtration efficiency in a subsequent filtration process for removing foreign matter from the aqueous composition for a hard capsule.

In the step S5, the plasticizer may be additionally added to the resulting solution in addition to the gelation agent and the gelation auxiliary agent.

At least one of the steps S1 to S5 may be performed while stirring.

After the step S5, the method may further include removing bubbles from the aqueous composition for a hard capsule.

The functions, types and concentrations of the alcohol, the water-soluble cellulose ether, the gelation agent, the gelation auxiliary agent and the plasticizer are as described above, and thus detailed description thereof are omitted.

However, the method for preparing an aqueous composition for hard capsule according to one exemplary embodiment of the present invention is not limited thereto, and may be widely modified by those skilled in the related art. For example, the cellulose ether solution prepared through the steps S1 to S3 may also be prepared through the following steps M1 to M3 or step N1.

That is, the cellulose ether solution may be prepared by mixing water and an alcohol to prepare an aqueous alcohol solution (M1), adding cellulose ether to the aqueous alcohol solution to prepare a cellulose ether solution (M2), and heating the cellulose ether solution to 40 to 70°C (M3).

Also, the cellulose ether solution may be prepared by mixing all of water, a water-soluble cellulose ether and an alcohol, each of which are heated to 40 to 70°C, to prepare a cellulose ether solution (N1).

According to the present invention, a hard capsule produced using the aqueous composition for a hard capsule is provided. For example, the hard capsule may be produced by dipping a mold pin kept at room temperature (20 to 30°C) into the aqueous composition for a hard capsule heated to a high temperature (40 to 70°C), removing the mold pin from the aqueous composition, and then drying the aqueous composition on the mold pin (this process is referred to as a 'cold pin process').

Hereinafter, the present invention will be described in further detail with reference to examples thereof. However, it should be understood that the description proposed herein is not intended to limit the scope of the present invention. The scope is defined by the claims.

### Example 1

15 parts by weight of ethanol and 65 parts by weight of purified water were mixed to prepare an aqueous ethanol solution. Thereafter, the aqueous ethanol solution was heated to 60°C, and 20 parts by weight of hydroxypropyl methyl cellulose (HPMC) (Samsung Fine Chemicals Co., Ltd., AW4) was then added to the aqueous ethanol solution, dissolved therein, and aged for 4 hours to prepare a cellulose ether solution. Then, 0.5 parts by weight (exclusive) of K-carrageenan as a gelation agent, and 0.5 parts by weight (exclusive) of potassium chloride as a gelation auxiliary agent were added, based on 100 parts by weight of the cellulose ether solution, to prepare an aqueous composition for a hard capsule. A test size #0 mold pin (commercially available from Technophar Equipment and Service Ltd.) was dipped into the aqueous composition for a hard capsule to produce a hard capsule.

### Examples 2 to 5 and Comparative Examples 1 and 2

Hard capsules were produced in the same manner as in Example 1, except that the concentrations of the K-carrageenan and the potassium chloride were adjusted as listed in the following Table 1.

### Comparative Example 3

80 parts by weight of purified water was heated to 80°C, and 20 parts by weight of HPMC (Samsung Fine Chemicals Co., Ltd., AW4) was dispersed, and then cooled to 50°C. Thereafter, the resulting dispersion was again heated to 60°C for 2 hours to prepare a solution, and the solution was then aged for 12 hours to prepare a cellulose ether solution. Subsequently, 1.0 part by weight (exclusive) of K-carrageenan as the gelation agent, and 0.5 parts by weight (exclusive) of potassium chloride as the gelation auxiliary agent were added, based on 100 parts by weight of the cellulose ether solution, to prepare an aqueous composition for a hard capsule. A test size #0 mold pin (commercially available from Technophar Equipment and Service Ltd.) was dipped into the aqueous composition for a hard capsule to produce a hard capsule.

### < Experimental Example 1: dissolution test >

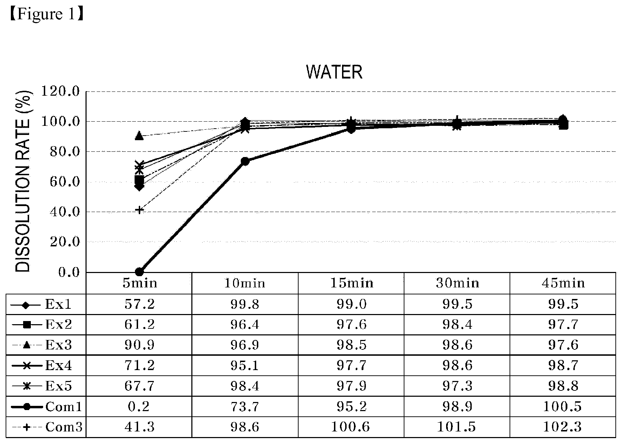

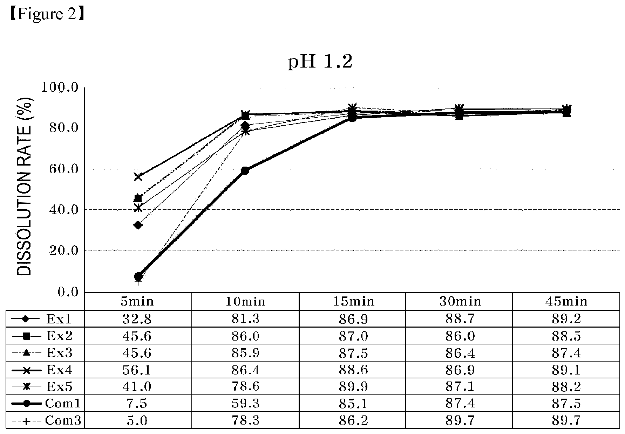

Each of the hard capsules prepared in Examples 1 to 5 and Comparative Examples 1 to 3 was filled with 300 mg of metformin, and a test was carried out using a dissolution testing system (Model Name: DT 1420, Maker: ERWEKA GmbH]. The dissolution test conditions were as follows: a temperature of 37°C, a rotation condition of 50 rpm, a test method such as a paddle method, and 900 ml of a medium (water, artificial gastric juice (pH 1.2), or artificial intestinal juice (pH 6.8)). The results are listed in Table 1. Also, the graphs illustrating dissolution rates of the hard capsules in the water and the artificial gastric or intestinal juices over time are shown in FIGS. 1 and 2.

**[Table 1]**

| | Concentration (parts by weight)* | | Dissolution rate (%) | | |
|---|---|---|---|---|---|
| | K-carrageenan | Potassium chloride | Water (5 minutes) | pH 1.2¹) (5 minutes) | pH 6.8²) (10 minutes) |
| Example 1 | 0.5 | 0.5 | 57.2 | 32.8 | 54.0 |
| Example 2 | 0.4 | 0.5 | 61.2 | 45.6 | 63.7 |
| Example 3 | 0.3 | 0.5 | 90.9 | 45.6 | 96.3 |
| Example 4 | 0.4 | 0.4 | 71.2 | 56.1 | 64.4 |
| Example 5 | 0.5 | 0.4 | 67.7 | 41.0 | 92.5 |
| Comparative Example 1 | 1.5 | 0.0 | 0.2 | 7.5 | 2.6 |
| Comparative Example 2 | 1.5 | 0.5 | 0.2 | 7.1 | 2.2 |
| Comparative Example 3 | 1.0 | 0.5 | 41.3 | 5.0 | 1.9 |

1) The artificial gastric juice (pH 1.2) was prepared by adding 7.0 ml of hydrochloric acid and water to 2.0 g of sodium chloride and adjusting to 1,000 ml according to a method of preparing an artificial gastric juice (pH 1.2) disclosed in the Korean Pharmacopoeia (KP).
2) The artificial intestinal juice (pH 6.8) was prepared by adding 118 ml of a 0.2 mol/L sodium hydroxide reagent and water and adjusting to 1,000 ml according to a method of preparing an artificial intestinal juice (pH 6.8) disclosed in the Korean Pharmacopoeia (KP).

Referring to Table 1, it was revealed that the hard capsules produced according to the present invention had a superior dissolution rate in water or at pH 1.2 or 6.8, compared to the hard capsules of Comparative Examples 1 to 3 including an excessive amount of the gelation agent.

Also, it can be seen that the hard capsules of Examples 1 to 5 produced according to the present invention had a significantly improved initial dissolution rate in water or at pH 1.2, compared to the hard capsules of Comparative Example 1 and 3, as shown in FIGS. 1 and 2.

While the invention has been described with reference to exemplary embodiments illustrated in accompanying drawings, these should be considered in a descriptive sense only, and it will be understood by those skilled in the art that various alterations and equivalent other embodiment may be made. Therefore, the scope of the invention is defined by the appended claims.

## Claims

1. An aqueous composition for a hard capsule comprising a water-soluble cellulose ether, an alcohol, and water,
wherein the aqueous composition for a hard capsule further comprises a gelation agent at greater than 0 parts by weight and not more than 0.5 parts by weight, and a gelation auxiliary agent at 0.3 to 0.6 parts by weight, based on 100 parts by weight of the aqueous composition for a hard capsule,
wherein the gelation agent comprises at least one water-soluble gum selected from the group consisting of carrageenan, gellan gum, xanthan gum, and pectin, and
wherein the gelation auxiliary agent comprises at least one selected from the group consisting of potassium chloride, potassium acetate, and calcium chloride.

2. The aqueous composition for a hard capsule of claim 1, wherein a concentration of the gelation agent is in a range of 0.3 to 0.5% by weight, based on 100 parts by weight of the aqueous composition for a hard capsule.

3. The aqueous composition for a hard capsule of claim 1, wherein a concentration of the water-soluble cellulose ether is in a range of 10 to 25% by weight, based on 100% by weight of the aqueous composition for a hard capsule.

4. The aqueous composition for a hard capsule of claim 1, wherein a concentration of the alcohol is in a range of 5 to 30% by weight, based on 100% by weight of the aqueous composition for a hard capsule.

5. The aqueous composition for a hard capsule of claim 1, wherein the water-soluble cellulose ether comprises at least one selected from the group consisting of hydroxypropyl methyl cellulose (HPMC), hydroxyethyl methyl cellulose (HEMC), and methyl cellulose (MC).

6. The aqueous composition for a hard capsule of claim 1, wherein the alcohol comprises at least one selected from the group consisting of ethanol, methanol, isopropanol, and butanol.

7. The aqueous composition for a hard capsule of claim 1, further comprising a plasticizer at 0.05 to 5.0 parts by weight, based on 100 parts by weight of the aqueous composition for a hard capsule.

8. The aqueous composition for a hard capsule of claim 7, wherein the plasticizer comprises at least one selected from the group consisting of glycerol, sorbitol, propylene glycol, and polyethylene glycol.

9. A hard capsule produced using the aqueous composition for a hard capsule defined in any one of claims 1 to 8.

## Patentansprüche

1. Wässrige Zusammensetzung für eine Hartkapsel, umfassend einen wasserlöslichen Celluloseether, einen Alkohol und Wasser,
wobei die wässrige Zusammensetzung für eine Hartkapsel ferner mit mehr als 0 Gewichtsanteilen und nicht mehr als 0,5 Gewichtsanteilen ein Geliermittel und mit 0,3 bis 0,6 Gewichtsanteilen ein Geliermittelhilfsmittel umfasst, basierend auf 100 Gewichtsanteilen der wässrigen Zusammensetzung für eine Hartkapsel,
wobei das Geliermittel mindestens einen wasserlöslichen Gummi umfasst, ausgewählt aus der Gruppe, bestehend aus Carrageenan, Gellangummi, Xanthangummi und Pektin, und
wobei das Geliermittelhilfsmittel mindestens eines umfasst, ausgewählt aus der Gruppe, bestehend aus Kaliumchlorid, Kaliumacetat und Calciumchlorid.

2. Wässrige Zusammensetzung für eine Hartkapsel nach Anspruch 1, wobei eine Konzentration des Geliermittels in einem Bereich von 0,3 bis 0,5 Gew.-% liegt, basierend auf 100 Gewichtsanteilen der wässrigen Zusammensetzung für eine Hartkapsel.

3. Wässrige Zusammensetzung für eine Hartkapsel nach Anspruch 1, wobei eine Konzentration des wasserlöslichen Celluloseethers in einem Bereich von 10 bis 25 Gew.-% liegt, basierend auf 100 Gew.-% der wässrigen Zusammensetzung für eine Hartkapsel.

4. Wässrige Zusammensetzung für eine Hartkapsel nach Anspruch 1, wobei eine Konzentration des Alkohols in einem Bereich von 5 bis 30 Gew.-% liegt, basierend auf 100 Gew.-% der wässrigen Zusammensetzung für eine Hartkapsel.

5. Wässrige Zusammensetzung für eine Hartkapsel nach Anspruch 1, wobei der wasserlösliche Celluloseether mindestens einen umfasst, ausgewählt aus der Gruppe, bestehend aus Hydroxypropylmethylcellulose (HPMC), Hydroxyethylmethylcellulose (HEMC) und Methylcellulose (MC).

6. Wässrige Zusammensetzung für eine Hartkapsel nach Anspruch 1, wobei der Alkohol mindestens einen umfasst, ausgewählt aus der Gruppe, bestehend aus Ethanol, Methanol, Isopropanol und Butanol.

7. Wässrige Zusammensetzung für eine Hartkapsel nach Anspruch 1, ferner umfassend mit 0,05 bis 5,0 Gewichtsanteilen einen Weichmacher, basierend auf 100 Gewichtsanteilen der wässrigen Zusammensetzung für eine Hartkapsel.

8. Wässrige Zusammensetzung für eine Hartkapsel nach Anspruch 7, wobei der Weichmacher mindestens einen umfasst, ausgewählt aus der Gruppe, bestehend aus Glycerol, Sorbitol, Propylenglykol und Polyethylenglycol.

9. Hartkapsel, hergestellt unter Verwendung der wässrigen Zusammensetzung für eine Hartkapsel nach einem der Ansprüche 1 bis 8.

## Revendications

1. Composition aqueuse pour une capsule dure comprenant un éther de cellulose soluble dans l'eau, un alcool, et de l'eau,
laquelle composition aqueuse pour une capsule dure comprend en outre un agent de gélification à raison de plus de 0 partie en poids et pas plus de 0,5 partie en poids, et un agent auxiliaire de gélification à raison de 0,3 à 0,6 partie en poids, pour 100 parties en poids de la composition aqueuse pour une capsule dure,
dans laquelle l'agent de gélification comprend au moins une gomme soluble dans l'eau choisie dans le groupe constitué par la carraghénane, la gomme gellane, la gomme xanthane, et la pectine, et
dans laquelle l'agent auxiliaire de gélification comprend au moins l'un choisi dans le groupe constitué par le chlorure de potassium, l'acétate de potassium, et le chlorure de calcium.

2. Composition aqueuse pour une capsule dure selon la revendication 1, dans laquelle la concentration de l'agent de gélification est située dans la plage allant de 0,3 à 0,5 % en poids, pour 100 parties en poids de la composition aqueuse pour une capsule dure.

3. Composition aqueuse pour une capsule dure selon la revendication 1, dans laquelle la concentration de l'éther de cellulose soluble dans l'eau est située dans la plage allant de 10 à 25 % en poids, pour 100 % en poids de la composition aqueuse pour une capsule dure.

4. Composition aqueuse pour une capsule dure selon la revendication 1, dans laquelle la concentration de l'alcool est située dans la plage allant de 5 à 30 % en poids, pour 100 % en poids de la composition aqueuse pour une capsule dure.

5. Composition aqueuse pour une capsule dure selon la revendication 1, dans laquelle l'éther de cellulose soluble dans l'eau comprend au moins l'un choisi dans le groupe constitué par l'hydroxypropylméthylcellulose (HPMC), l'hydroxyéthylméthylcellulose (HEMC) et la méthylcellulose (MC).

6. Composition aqueuse pour une capsule dure selon la revendication 1, dans laquelle l'alcool comprend au moins l'un choisi dans le groupe constitué par l'éthanol, le méthanol, l'isopropanol, et le butanol.

7. Composition aqueuse pour une capsule dure selon la revendication 1, comprenant en outre un plastifiant à raison de 0,05 à 5,0 parties en poids pour 100 parties en poids de la composition aqueuse pour une capsule dure.

8. Composition aqueuse pour une capsule dure selon la revendication 7, dans laquelle le plastifiant comprend au moins l'un choisi dans le groupe constitué par le glycérol, le sorbitol, le propylèneglycol, et le polyéthylèneglycol.

9. Capsule dure produite par utilisation de la composition aqueuse pour une capsule dure définie dans l'une quelconque des revendications 1 à 8.
